# EUROPÄISCHE PATENTSCHRIFT

(11) **EP 0 962 524 B1**
(45) Veröffentlichungstag und Bekanntmachung des Hinweises auf die Patenterteilung: **03.11.2004**
(21) Anmeldenummer: 99107819.7
(22) Anmeldetag: 20.04.1999
(51) Int. Cl.: C12M 3/00, C12N 15/89

(54) **Verfahren und Vorrichtung zur intrazellulären Manipulation einer biologischen Zelle**
Apparatus and process for the intracellular manipulation of a biological cell
Appareil et procédé pour la manipulation intracellulaire d'une cellule biologique

(30) Priorität: 27.05.1998 DE 19823655; 23.06.1998 DE 29811066 U; 10.09.1998 DE 19841337
(43) Veröffentlichungstag der Anmeldung: 08.12.1999
(73) Patentinhaber: Micronas GmbH, 79108 Freiburg (DE)
(72) Erfinder: Baumann, Werner, Dr., 79100 Freiburg (DE); Ehret, Ralf, Dr., 79291 Merdingen (DE); Lehmann, Mirko, Dipl.-Phys., 79117 Freiburg (DE); Igel, Günter, Dipl.Ing., 79331 Teningen (DE); Gahle, Hans-Jörg, Dr., 79312 Emmendingen (DE); Wolf, Bernhard, Prof. Dr., 79252 Stegen (DE); Sieben, Ulrich, Dr., 79276 Reute (DE); Freund, Ingo, Dipl.-Ing., 79235 Vogtsburg-Oberrottweil (DE); Brischwein, Martin Dr., 97424 Schweinfurt (DE)
(74) Vertreter: Bickel, Michael

(56) Entgegenhaltungen:
- EP-A- 0 292 899
- EP-A- 0 539 660
- WO-A-97/17426
- DE-A- 4 004 198
- DE-A- 4 031 138
- DE-A- 4 400 955
- US-A- 5 262 128
- US-A- 5 457 041

## Beschreibung

Die Erfindung betrifft ein Verfahren zur intrazellulären Manipulation wenigstens einer in einem Nährmedium befindlichen, adhärent an einem Auflagebereich angelagerten biologischen Zelle, wobei in die Zellmembran der Zelle eine Öffnung eingebracht und das Zellinnere durch diese Öffnung hindurch manipuliert wird. Die Erfindung bezieht sich ferner auf eine Vorrichtung zur Manipulation des Zellinneren wenigstens einer in einem Nährmedium befindlichen, eine Zellmembran aufweisenden biologischen Zelle, mit einem Objektträger, der wenigstens einen Auflagebereich hat, an dem die Zelle adhärent anlagerbar ist, mit einem Porationswerkzeug zum Öffnen der Zellmembran, und mit wenigstens einem im Bereich des Porationswerkzeugs befindlichen Durchtrittskanal zur Manipulation des Zellinneren.

Aus Alberts, B. et al., Molekularbiologie der Zelle, 3. Auflage, VCH-Verlag (1995), Seite 212 ff., kennt man bereits eine Vorrichtung der eingangs genannten Art, die eine mit einer Absaugvorrichtung verbundene, aus einem elektrisch isolierenden Material bestehende Hohlnadel mit einer Innenhöhlung aufweist, die am freien Ende der Hohlnadel eine Öffnung hat. Zum Öffnen der Zellmembran wird die am freien Ende der Hohlnadel befindliche Öffnung außenseitig an die Zellmembran angesetzt, um dann mittels der Absaugvorrichtung einen Unterdruck in der Innenhöhlung der Hohlnadel zu erzeugen. Durch diesen Unterdruck wird ein vor der Öffnung der Hohlnadel befindliches Zellmembranstück aus dem Membranverband herausgerissen. Nach dem Einbringen der Öffnung in die Zellmembran isoliert die an dem die Öffnung umgrenzenden Randbereich der Zellmembran angreifende Holnadel die im Inneren der Zelle enthaltene Zellflüssigkeit elektrisch gegen das Nährmedium. Durch die in die Zellmembran eingebrachte Öffnung hindurch wird dann das Innere der Zelle manipuliert. Beispielsweise kann der Zellkern durch Ansaugen von Zellflüssigkeit in die Hohlnadel aus der Zelle entfernt werden und anschließend ein ander Zellkern durch die Öffnung in das Zellinnere eingebracht werden.

Das vorbekannte Verfahren und die für Durchführung des Verfahrens verwendete Vorrichtung haben den Nachteil, daß zum Positionieren der Hohlnadel an der Zelle ein Mikromanipulator erforderlich ist. Dadurch ergibt sich eine vergleichsweise komplizierte und teure Vorrichtung. Außerdem wird die Zugänglichkeit der auf dem Objektträger befindlichen Zellen durch den Mikromanipulator stark eingeschränkt. Das Verfahren und die Vorrichtung eignen sich deshalb nur für eine intrazelluläre Manipulation einzelner oder allenfalls für eine gleichzeitige Manipulation einer kleinen Anzahl auf dem Objektträger befindlicher Zellen. Dabei ist ein aufwendiges manuelles Positionieren der Hohlnadel an der Zelle erforderlich.

Aus Alberts, B. et al., Molekularbiologie der Zelle, 3. Auflage, VCH-Verlag (1995), Seite 213 ist auch bereits bekannt, eine in einem Nährmedium schwimmende Zelle zwischen zwei jeweils von der Zelle beabstandeten großflächigen Elektroden anzuordnen und an diese Elektroden eine elektrische Spannung anzulegen. Dabei wird die Zellmembrane der Zelle durch Elektroporation an mehreren Stellen gleichzeitig geöffnet, so daß in dem Nährmedium befindliches, in die Zelle einzubringendes Genmaterial durch die Öffnungen der Zelle hindurch in das Innere der Zelle diffundieren können. Nachteilig ist dabei jedoch, daß eine solche Einbringung von Genmaterial statistischen Schwankungen unterliegt und von verschiedenen Parametern beeinflußt wird, wie beispielsweise der Konzentration des Genmaterials im Bereich der Zelle, der Größe des einzubringenden Genmaterials und der Größe der in die Zellmembran eingebrachten Öffnungen. Das vorbekannte Verfahren ermöglich somit keine gezielte Manipulation des Zellinneren.

Es besteht deshalb die Aufgabe, ein Verfahren und eine Vorrichtung der eingangs genannten Art zu schaffen, die ein einfaches Manipulieren des Inneren einer Zelle ermöglichen. Insbesondere soll ein aufwendiges manuelles Positionieren einer Hohlnadel an der zu behandelnden Zelle vermieden werden.

Die Lösung dieser Aufgabe besteht bezüglich des Verfahrens darin, daß die Öffnung innerhalb des Auflagebereichs der Zelle und mit Abstand von deren Auflagerand in die Zellmembrane eingebracht wird.

Dadurch ist es möglich, ein für das Einbringen der Zellmembran-Öffnung verwendetes Porationsmittel oder ein Porationswerkzeug in dem Auflagebereich der Zelle an dem Objektträger anzuordnen, so daß die Zelle beim Anlagern an dem Auflagebereich gleichzeitig auch an dem Porationsmittel oder dem Porationswerkzeug positioniert ist. Dadurch kann ein aufwendiges manuelles Positionieren eines Porationswerkzeuges entfallen. Da die Öffnung innerhalb des Auflagebereichs der Zelle und mit Abstand vom Rand des Auflagebereichs in die Zellmembran eingebracht wird, dichtet der die Öffnung umgrenzende, adhärent an dem Auflagebereich anhaftende Membranbereich der Zelle die Öffnung gegen das Nährmedium ab. Die im Inneren der Zelle befindliche Zellflüssigkeit ist dadurch elektrisch weitestgehend gegen das Nährmedium isoliert, so daß ein Potentialausgleich zwischen dem Zellpotential im Inneren der Zelle und dem des Nährmediums verhindert wird. Durch die in die Zellmembran eingebrachte Öffnung hindurch kann dann das Zellinnere manipuliert werden.

Bei einer besonders vorteilhaften Ausführungsform der Erfindung wird die Öffnung mittels Elektroporation in die Zellmembran eingebracht. Bei der Durchführung des Verfahrens kann beispielsweise eine Elektroporations-Elektrode in dem Auflagebereich für die Zelle angeordnet werden, an der sich die Zelle adhärent anlagert. Zum Einbringen der Öffnung in die Zellmembran braucht dann nur noch eine elektrische Spannung zwischen der Elektroporations-Elektrode und dem Nährmedium angelegt zu werden, die einen elektrischen Stromfluß bewirkt, der die Zellmembran öffnet.

Bei einer anderen Ausführungsform der Erfindung wird zum Einbringen der Öffnung in die Zellmembran auf einen Teilbereich der Zellmembran wenigstens ein mechanischer Impuls ausgeübt. Dabei löst sich dieser Teilbereich der Zellmembran aus dem Membranverbund heraus. Gegebenenfalls kann auch eine Impulsfolge mit mehreren Einzelimpulsen zur Anwendung kommen.

Besonders vorteilhaft ist, wenn die Öffnung mittels Schallwellen, insbesondere Ultra- und/oder Hyperschallwellen in die Zellmembran eingebracht wird. Dabei ist es sogar möglich, daß die Schallwellen auf den zu öffnenden Bereich der Zellmembran fokussiert wird und/oder daß mehrere Schallwellen derart überlagert werden, daß sich ihre Schwingungen in dem zu öffnenden Bereich der Zellmembran zu einer Schwingung mit erhöhter Amplitude überlagern. Die Zellmembran kann dadurch berührungslos geöffnet werden.

Eine berührungslose Öffnung der Zellmembran kann aber auch in der Weise erfolgen, daß ein Teilbereich der Zellmembran mit energiereicher Strahlung, insbesondere mit Laserstrahlung bestrahlt wird. Dabei wird die Wellenlänge der Strahlung vorzugsweise so gewählt, daß die Zellmembran die Strahlung gut absorbiert. Zweckmäßigerweise wird die Strahlung an dem Auflagebereich der Zelle in diese eingekoppelt. Gegebenenfalls kann ein Laserstrahl aber auch außerhalb des Auflagebereichs in die Zelle eingekoppelt werden, indem in einen dort befindlichen Membranbereich zunächst eine kleine Einkoppelöffnung eingebracht wird, durch die der Laserstrahl anschließend durch das Innere der Zelle hindurch auf einen im Auflagebereich der Zelle befindlichen Membranbereich projiziert wird, um dort durch Verschwenken des Laserstrahls um die Einkoppelöffnung einen Teilbereich der Membran aus dem Membranverbund herauszuschneiden.

Bei einer anderen Ausführungsform des Verfahrens wird die Öffnung durch Einwirkung einer chemischen Porations-Substanz in die Zellmembran eingebracht. Als Porationsmittel kann dabei beispielsweise ein Perforin oder Triton® verwendet werden.

Besonders vorteilhaft ist, wenn eine elektrisch und/oder chemisch und/oder durch Strahlung aktivierbare chemische Substanz verwendet wird und wenn diese Substanz zum Einbringen der Öffnung in die Zellmembran durch Einwirkung von Strahlung, einer Chemikalie und/oder eines elektrischen Feldes aktiviert wird. Die Substanz wird also durch Energiezufuhr aktiviert. Dabei können zum Beispiel freie Radikale erzeugt werden, welche den zu öffnenden Teilbereich der Zellmembran zerstören. Im inaktiven Zustand verhält sich die Substanz gegenüber der Zelle weitgehend neutral, so daß sie das Anlagern der Zelle an dem Auflagebereich praktisch nicht beeinflußt. Es kann auch eine chemische Substanz verwendet werden, die durch Zugabe einer weiteren Substanz chemisch aktiviert wird.

Eine andere Ausführungsform des Verfahrens sieht vor, daß ein zu öffnender Teilbereich der Zellmembran durch Beaufschlagung mit einem Unterdruck und/oder einem Überdruck aus dem Membranverband herausgelöst wird. Dazu kann beispielsweise in einem den Auflagebereich aufweisenden Objektträger innerhalb des Auflagebereichs eine kleine Öffnung vorgesehen sein, durch welche die Zelle so stark angesaugt wird, daß der vor der Öffnung befindliche Membranbereich aus dem Membranverbund herausgerissen wird. Zum Öffnen der Zellmembran kann durch die Öffnung hindurch aber auch ein Überdruckimpuls auf einen Membranbereich der Zelle ausgeübt werden.

Vorteilhaft ist, daß die Zelle durch eine Saugkraft an dem Auflagebereich fixiert wird. Dadurch kann das Anhaften der Zelle an dem Auflagebereich eines Objektträgers verbessert werden. Die Saugkraft ist dabei so bemessen, daß die Zellmembran durch die Saugkraft mechanisch nicht beschädigt wird.

Vorteilhaft ist, wenn nach dem Einbringen der Öffnung in die Zellmembran durch die Öffnung hindurch wenigstens eine Substanz und/oder ein Zellbestandteil aus dem Zellinneren entnommen und/oder in das Zellinnere eingebracht wird. So kann beispielsweise ein Medikament, ein Protein und/oder eine andere biologisch wirksame Substanz durch die Öffnung hindurch in das Innere der Zelle gebracht werden, um deren Reaktion zu testen. Es kann aber auch ein Gen oder ein Genfragment aus der Zelle entnommen oder in diese eingebracht werden. Es kann sogar der Zellkern durch die Öffnung hindurch aus der Zelle entnommen und durch einen anderen ersetzt werden.

Bezüglich der Vorrichtung besteht die Lösung der vorstehend genannten Aufgabe darin, daß das Porationswerkzeug innerhalb des Auflagebereichs angeordnet ist. Die Zelle kann sich dadurch an dem in dem Auflagebereich befindlichen Porationswerkzeug anlagern. In vorteilhafter Weise wird dadurch ein aufwendiges manuelles Positionieren des Porationswerkzeuges an der Zelle vermieden. Auch werden keine Hilfsvorrichtungen, wie beispielsweise Mikromanipulatoren benötigt. Dadurch ist es möglich, mehrere dicht benachbart zu einander in dem Auflagebereich angeordnete Zellen gleichzeitig intrazellulär zu manipulieren. Nach dem Einbringen der Öffnung in die Zellmembran verbleibt der die Öffnung umgebene Rand der Zellmembran mit dem Auflagebereich des Objektträgers in Berührung und dichtet die Öffnung der Zellmembrane gegen das Nährmedium ab.

Eine besonders vorteilhafte Ausführungsform der Erfindung sieht vor, daß das Porationswerkzeug innerhalb des Auflagebereichs von wenigstens einem elektrischen Isolator umgrenzt ist. Durch den elektrischen Isolator ist die im Inneren der Zelle befindliche Zellflüssigkeit dann elektrisch gut gegen das Nährmedium isoliert, so daß das Innere der lebenden Zelle über einen längeren Zeitraum manipuliert werden kann, ohne daß die Zelle abstirbt. Der Isolationswiderstand ist abhängig vom Zelltyp und ist vorzugsweise größer als 10 Megaohm. Dadurch wird ein Potentialausgleich zwischen dem Nährmedium und der Zellflüssigkeit weitestgehend unterbunden. Gegebenenfalls kann der Objektträger vollständig aus einem isolierenden Material bestehen. Der Isolator kann aber auch im Inneren des Objektträgers mit Abstand zur Oberfläche des Auflagebereichs angeordnet sein.

Vorteilhaft ist, wenn das Porationswerkzeug die im Auflagebereich des Objektträgers befindliche Mündung des Durchtrittskanals im wesentlichen konzentrisch umgrenzt. Das Zellinnere kann dann durch die Öffnung hindurch noch besser manipuliert werden, ohne daß bei der Manipulation der die Öffnung umgrenzende Randbereich der Zellmembran beschädigt wird.

Besonders vorteilhaft ist, wenn das Porationswerkzeug eine Elektroporations-Elektrode ist, der eine mit dem Nährmedium in Kontakt bringbare Referenzelektrode zugeordnet ist, und wenn die Elektroporations-Elektrode und die Referenzelektrode zum Elektroporieren der Zellmembran der Zelle mit einer elektrischen Spannungsquelle verbindbar sind. Die Elektroporations-Elektrode ist innerhalb des Auflagebereichs des Objektträgers angeordnet, so daß sich eine an dem Isolator adhärent angelagerte Zelle gegebenenfals an der Elektrode anlagern kann oder sich zumindest bis in den Wirkungsbereich eines von der Elektrode ausgehenden elektrischen Feldes an diese annähern kann. Beim Anlegen der Elektroporations-Spannung an die Elektrode fließt ein elektrischer Strom, der in die Zellmembran eine Öffnung einbringt. Dabei verbleibt der die Öffnung umgebene Rand der Zellmembran mit dem Auflagebereich in Berührung und dichtet die Öffnung gegen das Nährmedium ab. Nach dem Einbringen der Öffnung in die Zellmembran wird die Elektrode von der Elektroporations-Spannungsquelle getrennt, so daß das Innere der Zelle dann durch die Öffnung hindurch verändert werden kann.

Bei einer vorteilhaften Ausführungsform der Vorrichtung ist das Porationswerkzeug zum Öffnen der Zellmembran der Zelle mittels wenigstens eines Aktuators, insbesondere eines Piezoelements quer zur Oberfläche des Auflagebereichs relativ zu dem Objektträger bewegbar. Bei dieser Vorrichtung wird die Öffnung also mechanisch in die Zellmembran eingebracht. Dabei kann das Porationswerkzeug gegebenenfalls wechselweise auf die Zellmembran zu und von dieser wegbewegt werden. Zu diesem Zweck kann der Aktuator mit einer Ansteuereinrichtung zum Erzeugen einer mechanischen Schwingung, insbesondere einer Ultra- oder Hyperschallschwingung verbunden sein. Das Porationswerkzeug kann in einer rechtwinklig oder in einer schräg zur Oberfläche des Auflagebereichs verlaufenden Richtung relativ zu dem Objektträger bewegbar sein.

Vorteilhaft ist, wenn das Porationswerkzeug mindestens eine, vorzugsweise gegenüber der Oberflächenebene des Auflagebereichs vorstehende scharfe Spitze oder Kante aufweist. Das Poratationswerkzeug kann dann gegebenenfalls an der Zellmembran anliegen, so daß diese noch besser mechanisch geöffnet werden kann. Wenn das Porationswerkzeug eine Elektrode zum Elektroporieren der Zellmembrane ist, ergibt sich beim Anlegen einer Porationsspannung an die Elektrode an der scharfen Spitze oder Kante eine besonders hohe elektrische Feldstärke, was das Öffnen der Zellmembrane erleichert.

Besonders vorteilhaft ist, wenn als Porationswerkzeug ein Laserstrahl vorgesehen ist und wenn der Strahlengang des Laserstrahls durch den Durchtrittskanal hindurch zu der im Auflagebereich befindlichen Mündung geführt ist. Mit dieser Vorrichtung kann ein Teilbereich der Zellmembran kurzzeitig mit energiereicher optischer Strahlung bestrahlt werden, wobei sich dieser so stark erwärmt, daß sich die Zellmembran öffnet. Gegebenenfalls können innerhalb oder außerhalb des Durchtrittskanals Strahlführungsmittel angeordnet sein.

Besonders vorteilhaft ist, wenn zum Erzeugen des Laserstrahls eine Laserdiode in den Objektträger integriert ist. Dabei kann die Laserdiode sogar direkt hinter der Mündung des Durchtrittskanals angordnet sein, so daß die Laserstrahlung unmittelbar und somit weitgehend verlustfrei in die Zellmembran der an dem Auflagebereich angelagerten Zelle eingekoppelt werden kann.

Bei einer vorteilhaften Ausführungsform ist vorgesehen, daß das Porationswerkzeug zum Öffnen der Zellmembran der Zelle im Auflagebereich des Objektträgers eine chemische Porations-Substanz aufweist und/oder daß eine chemische Porations-Substanz durch den Durchtrittskanal zu dessen Mündung zuführbar ist. Die Öffnung kann also auch chemisch in die Zellmembran eingebracht werden, wobei als Porationsmittel zum Beispiel Perforine oder Triton® verwendet werden können. Gegebenenfalls kann der Durchtrittskanal mit einem die Porations-Substanz enthaltenden Depot verbunden oder verbindbar sein, aus dem die Porations-Substanz der Zellmembran zuführbar ist.

Bei einer anderen Ausführungsform weist die Vorrichtung wenigstens eine Pumpe auf, die zum Öffnen der Zellmembran durch Beaufschlagung mit Unter- oder Überdruck mit dem Durchtrittskanal verbunden ist und/oder daß in der Durchtrittskanal über ein Ventil oder dergleichen Absperrelement mit einem Unter- oder Überdruckspeicher verbindbar ist. Die Öffnung kann dann durch Unter- oder Überdruck in die Zellmembran eingebracht werden, wobei der Unter- bzw. Überdruck nach dem Öffnen der Zellmembran abgeschaltet wird. Dadurch wird bei einem Öffnen der Zellmembran durch Unterdruck ein Absaugen von Zellflüssigkeit aus dem Inneren der Zelle weitestgehend vermieden. Entsprechend wird beim Öffnen der Zellmembran mittels Überdruck vermieden, daß ein in dem Durchtrittskanal befindliches Medium, das vorzugsweise ein Fluid ist, in das Innere der Zelle gelangen kann. Zum Abschalten des Unter- bzw. Überdrucks kann beispielseise eine beim Öffnen der Zellmembran in dem Durchtrittskanal auftretende Druckveränderung ermittelt werden. In vorteilhafter Weise kann der Durchtrittskanal vor dem Anlagern der Zelle auch dazu genutzt werden, um Nährmedium aus dem Auflagebereich abzusaugen, so daß in dem Nährmedium eine Strömung entsteht, welche die darin befindlichen Zellen zu der im Bereich des Porationswerkzeugs angeordneten Mündung des Durchtrittskanals leitet.

Eine vorteilhafte Ausführungsform sieht vor, daß das Porationswerkzeug an einem gegenüber der Oberflächenebene des Auflagebereichs vorstehenden Vorsprung angeordnet ist. Dadurch ergibt sich ein guter elektrischer und/oder mechanischer Kontakt zwischen dem Porationswerkzeug und der Zellmembran.

Zweckmäßigerweise ist vorgesehen, daß sich der Querschnitt des Vorsprungs ausgehend von der Oberflächenebene des Auflagebereichs zu der am weitesten vorstehenden Stelle verjüngt. Die Zelle haftet dann im Bereich des Vorsprungs besonders gut an dem Auflagebereich des Objektträgers an. Außerdem kann der Isolator bei der Herstellung des Objektträgers als Beschichtung fertigungstechnisch besser auf dem sich verjüngenden Bereich des Vorsprungs aufgetragen werden.

Bei einer vorteilhaften Weiterbildung der Erfindung ist vorgesehen, daß der Objektträger im Auflagebereich eine Profilierung aufweist, die wenigstens eine um das Porationswerkzeug umlaufende Profilierungsvertiefung und/oder einen um das Porationswerkzeug umlaufenden Profilierungsvorsprung hat. Dadurch wird eine bessere Abdichtung der Zellflüssigkeit gegen das Nährmedium durch die an dem Isolator anhaftende Zellmembran erreicht.

Vorteilhaft ist, wenn die Profilierungsvertiefung und/oder der Profilierungsvorsprung in Erstreckungsrichtung durch wenigstens eine Unterbrechung unterbrochen ist. Die Zelle kann dann im Bereich der Profilierung besser an der Oberfläche des Objektträgers anhaften. Der Profilierungsvorsprung bzw. die Profilierungsvertiefung können beispielsweise eine Wabenstruktur oder eine Struktur nach Art eines Karo- oder Schachbrettmusters aufweisen.

Besonders vorteilhaft ist, wenn die Profilierungsvertiefung und/oder der Profilierungsvorsprung ringförmig ausgebildet ist und wenn vorzugsweise mehrere solcher ringförmiger Profilierungsvertiefungen und/oder Profilierungsvorsprünge im wesentlichen konzentrisch zum Porationswerkzeug angeordnet sind. Somit sind radial zum Porationswerkzeug mehrere Profilierungsvertiefungen und/oder -vorsprünge hintereinander geschaltet bzw. ineinander verschachtelt, so daß die Zellflüssigkeit noch besser gegen das Nährmedium abgedichtet ist.

Eine bevorzugte Ausführungsform der Erfindung sieht vor, daß der elektrische Isolator eine an der Oberfläche der Profilierung angeordnete Isolationsschicht ist. In vorteilhafter Weise wird durch die so profilierte Isolationsschicht der Weg für einen an der Oberfläche des Isolators von der Zellflüssigkeit zu dem Nährmedium fließenden Kriechstrom vergrößert, so daß die im Inneren der Zelle befindliche Zellflüssigkeit nach dem Öffnen der Zellmembran noch besser gegen das Nährmedium isoliert ist.

Eine andere Ausführungsform sieht vor, daß der (die) Profilierungsvorsprung (-vorsprünge) auf die Oberfläche des elektrischen Isolators aufgebracht ist (sind). Der Objektträger ist dann fertigungstechnisch einfacher herstellbar.

Besonders vorteilhaft ist, wenn daß im Auflagebereich des Objektträgers an dessen Oberfläche eine wenigstens ein Zelladhäsionsprotein aufweisende Beschichtung und/oder eine hydrophile Beschichtung und/oder unmittelbar benachbart zu dem Porationswerkzeug eine hydrophobe Beschichtung angeordnet ist. Die Zellmembran der Zelle haftet dann besser an dem Objektträger an. Die Zelladhäsions-Beschichtung kann beispielsweise Laminin, Fibronectin oder Poly-L-Lysin aufweisen. Gegebenenfalls kann an dem an die Elektrode angrenzenden Rand des Auflagebereichs auch eine hydrophobe Beschichtung mit Bindungsstellen für in der Zellmembran befindliche hydrophobe Lipide angeordnet sein.

Vorteilhaft ist, wenn als mechanische Führung für die Zellen beidseits des Porationswerkzeugs Begrenzungswände angeordnet sind, die vorzugsweise einen nutenartigen Führungskanal begrenzen. Dabei ist das Porationswerkzeug vorzugsweise mittig zwischen den Begrenzungswänden am Nutgrund des Führungskanal angeordnet, so daß in dem Führungskanal befindliche Zellen sich im wesentlichen nur in Erstreckungsrichtung des Führungskanals bewegen können und dann zwangsläufig mit dem Porationswerkzeug in Berührung geraten.

Vorteilhaft ist, wenn zum Erzeugen eines die Zelle zu dem Porationswerkzeug leitenden elektrischen Feldes im Auflagebereich und/oder benachbart dazu wenigstens eine Zusatzelektrode angeordnet ist. Dadurch kann an der Oberfläche des Objektträgers ein elektrisches Feld erzeugt werden, das auf biologische Zellen, deren Dielektrizitätskonstante sich von derjenigen des Nährmediums, in dem sie angeordnet sind, unterscheidet, eine Kraft ausübt, welche die Zellen zu dem Porationswerkzeug leitet.

Bei einer bevorzugten Ausführungsform der Erfindung ist der Objektträger als etwa plattenförmiges Trägerteil ausgebildet, das an einer Flachseite den Auflagebereich aufweist, daß der Durchtrittskanal den Objektträger ausgehend von dem Auflagebereich zu der diesem abgewandten Rückseite des Objektträgers durchsetzt, und daß der Objektträger im Bereich des Durchtrittskanals eine Wandungsschwächung aufweist, die in Erstreckungsrichtung des Durchtrittskanals eine kleinere Abmessung aufweist als ein zu der Wandungsschwächung benachbarter Wandungsbereich. Der Durchtrittskanal ist also im Bereich einer Wandungsschwächung durch den Objektträger hindurchgeführt und durchsetzt diesen vorzugsweise rechtwinklig zu der durch den Auflagebereich aufgespannten Ebene. Dadurch wird eine besonders kurze Durchtrittskanallänge erreicht. Der Durchtrittskanal kann beispielsweise mit einer an der Rückseite des Objektträgers angeordneten Pumpe oder einer Absaugeinrichtung verbunden sein. Der an die Wandungsschwächung angrenzende und diese vorzugsweise umgrenzende Wandungsbereich des Objektträgers weist eine größere Wandicke auf als die Wandungsschwächung, was die mechanische Stabilität des Objektträgers verbessert.

Vorteilhaft ist, wenn die Wandungsschwächung an einer an der dem Auflagebereich abgewandten Rückseite des Objektträgers befindlichen, vorzugsweise trichterförmigen Einformung angeordnet ist. Der Auflagebereich kann dann in einer sich bis über die Wandungsschwächung hinweg erstreckenden Ebene angeordnet sein, so daß sich die Zellen besser daran anlangern können.

Eine besonders vorteilhafte Weiterbildung der Erfindung sieht vor, daß in dem Auflagebereich mehrere, jeweils wenigstens einen Durchtrittskanal aufweisende Porationswerkzeuge vorzugsweise als Arrays angeordnet sind. Mit einer solchen Vorrichtung kann eine Vielzahl dicht zueinander benachbarter Zellen entweder gleichzeitig oder zeitlich nacheinander intrazellulär manipuliert werden, wodurch statistische Schwankungen eleminiert werden können. Gegebenenfalls kann in den Objektträger auch ein Multiplexer integriert sein, mit dem eine Vielzahl von Elektroporations-Elektroden wechselweise nacheinander angesteuert werden können, wodurch sich Anzahl der Zuleitungen zu dem als Objektträger entsprechend reduziert.

Nachfolgend sind Ausführungsbeispiele der Erfindung anhand der Zeichnung näher erläutert. Es zeigen:
- Fig.1: einen Längsschnitt durch eine Vorrichtung zur Manipulation des Zellinneren einer Zelle, mit einem ein Porationswerkzeug aufweisenden Objektträger, auf dem eine in einem Nährmedium befindliche biologische Zelle adhärent angelagert ist,
- Fig.2: einen Längsschnitt durch eine Vorrichtung ähnlich Fig.1, wobei jedoch der Auflagebereich des Objektträgers unprofiliert ist,
- Fig.3: eine Aufsicht auf das in Fig.2 gezeigte Porationswerkzeug,
- Fig.4: eine Darstellung ähnlich Fig.2, wobei jedoch das als Elektrode ausgebildete Porationswerkzeug eine im wesentlichen zylindrische Form aufweist,
- Fig.5: eine Aufsicht auf die Elektrode gemäß Fig.4,
- Fig.6: einen Längsschnitt durch eine Vorrichtung, bei der das Porationswerkzeug mittels eines Piezo-Elements bewegbar ist,
- Fig.7: einen Längsschnitt durch eine Vorrichtung, bei der ein Laserstrahl in den Durchtrittskanal eingekoppelt wird,
- Fig.8: einen Längsschnitt durch einen Objektträger, der eine zwischen zwei Begrenzungswänden innerhalb einer Oberflächenstrukturierung angeordnetes Porationswerkzeug aufweist,
- Fig.9: eine Aufsicht auf einen Objektträger, der ein Array mit einer Vielzahl von Porationswerkzeugen und Durchtrittskanälen aufweist,
- Fig.10: eine Aufsicht auf einen Objektträger, dessen Auflagebereich eine Karo-Strukturierung aufweist und
- Fig.11: eine Aufsicht auf einen Objektträger, dessen Auflagebereich wabenförmig strukturiert ist.

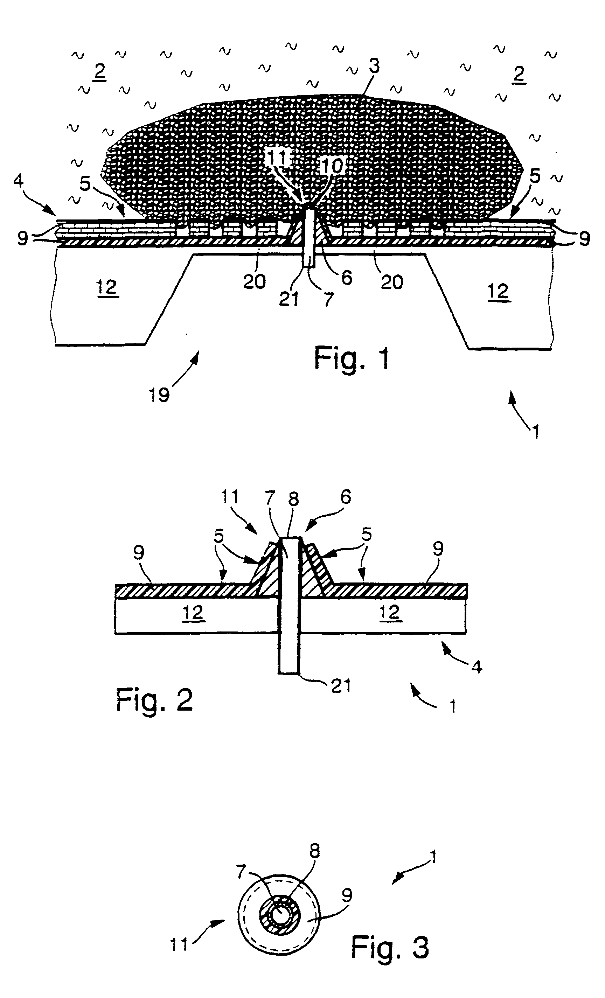

Eine im ganzen mit 1 bezeichnete Vorrichtung zur Manipulation des Zellinneren einer in einem Nährmedium 2 befindlichen biologischen Zelle 3 (Fig.1) weist einen Objektträger 4 auf, der einen Auflagebereich 5 hat, an den die Zelle 3 adhärent anlagerbar ist. Die Zelle 3 ist also auf dem Objektträger 4 immobilisiert und haftet an dem Auflagebereich 5 an. Zum Einbringen einer Öffnung in die an dem Auflagebereich 5 anhaftende Zelle 3 ist innerhalb des Auflagebereichs 5 ein Porationswerkzeug 6 angeordnet. Der Objektträger 4 weist einen Durchtrittskanal 7 zur Manipulation des Zellinneren auf, der im Bereich des Porationswerkzeugs 6 in den Auflagebereich 5 mündet. In Gebrauchsstellung ist der die Mündung umgrenzende Rand des Objektträgers 4 mit Abstand vom Auflagerand der Zelle 3 angeordnet ist. Der an dem Objektträger 4 anhaftende Membranbereich der Zelle 3 umgrenzt also die Mündung des Durchtrittskanals 7, wodurch die im Inneren der Zelle 3 befindliche Zellflüssigkeit nach dem Einbringen der Öffnung in die Zellmembran gegen das Nährmedium 2 abgedichtet wird.

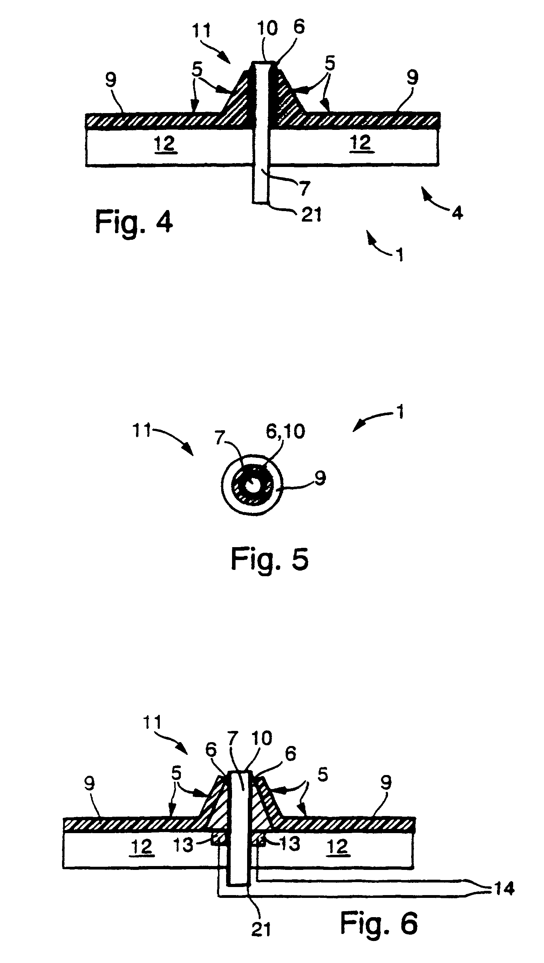

Bei den Ausführungsbeispielen nach Fig.1 bis 3 ist das Porationswerkzeug 6 eine Elektroporations-Elektrode, die einen gegenüber der Oberflächenebene des Auflagebereichs 5 vorstehenden aktiven Elektrodenbereich 8 hat. Die Elektrode ist als Hohl-Elektrode ausgebildet, die von dem Durchtrittskanal 7 durchsetzt ist. Ein Abschnitt des Durchtrittskanals 7 erstreckt sich von dem dem aktiven Elektrodenbereich 8 abgewandten Ende der Elektrode zu dem aktiven Elektrodenbereich 8. Wie aus Figur 3 und 5 besonders gut erkennbar ist, umgrenzt der aktive Elektrodenbereich 8 die Mündung des Durchtrittskanals 7 ringförmig. In dem Auflagebereich 5 ist ferner ein den aktiven Elektrodenbereich 8 umgrenzender elektrischer Isolator 9 angeordnet, an dem die Zelle 3 gegen das Nährmedium 2 abdichtend anlagerbar ist.

Die Elektrode mittels einer in den Objektträger integrierten Leiterbahn an ein elektrisches oder elektronisches Schaltelement angeschlossen, mit dem sie mit einer Elektroporations-Spannungsquelle verbindbar ist. Zum Öffnen der Zellmembran wird zwischen dem Porationswerkzeug 6 und dem Nährmedium 2 eine elektrische Spannung angelegt, woraufhin über das Porationswerkzeug 6 ein elektrischer Strom in die Zellmembran fließt, der die Zellmembran im Bereich des Porationswerkzeugs 6 öffnet. Die in die Zelle 3 mittels des Porationswerkzeugs 6 eingebrachte Öffnung ist durch den sie umschließenden, an dem Isolator 9 des Objektträgers 4 anhaftenden Zellmembranbereich gegen das Nährmedium 2 abgedichtet. Dadurch wird ein Potentialausgleich zwischen dem Potential der im Inneren der Zelle 3 befindlichen Zellflüssigkeit und dem des Nährmediums 2 verhindert.

Nach dem Öffnen der Zellmembran wird das Innere der Zelle 3 durch den Durchtrittskanal 7 hindurch manipuliert. Dabei kann beispielsweise durch den Durchtrittskanal 7 eine Substanz aus dem Zellinneren entnommen und/oder in das Zellinnere eingebracht werden. Die Substanz kann beispielsweise ein Zellbestandteil, ein Gen, ein Protein, ein toxischer Stoff, den die Zelle detektieren soll, und/oder ein Medikament, dessen Wirkung auf die Zelle untersucht oder mit dem die Zelle behandelt werden soll, aufweisen. Es können aber auch in der Zelle vorhandene Substanzen oder Zellbestandteile verändert werden, indem sie beispielsweise durch den Durchtrittskanal 7 hindurch mit energiereicher Strahlung bestrahlt werden. Durch den Durchtrittskanal 7 hindurch kann auch ein Mikromanipulator oder dergleichen Werkzeug in das Innere der Zelle eingeführt werden, um dort mechanisch das Zellinnere zu manipulieren. Dadurch kann an eienr bestimmten Stelle im Zellinneren gezielt eine Veränderung vorgenommen werden.

Bei den Ausführungsbeispielen nach Fig.1 bis 3 weist das als Elektrode ausgebildete Porationswerkzeug 6 etwa die Form eines Kegelstumpfs auf, wobei der im wesentlichen zylindrische Durchtrittskanal 7 die Elektrode entlang der Mittelachse des Kegels durchsetzt. Die Symmetrieachse der Elektrode ist jeweils etwa rechtwinklig zur Oberflächenebene des Objektträgers 4 im Auflagebereich 5 angeordnet. Das Porationswerkzeug 6 kann ganz oder teilweise in die Oberfläche des Objektträgers 4 eingelassen sein.

Der aktive Elektrodenbereich 8 der Elektrode umgrenzt jeweils die Mündung des Durchtrittskanals 7 und weist eine gegenüber der Oberflächenebene des Auflagebereichs 5 vorstehende, scharfe Ring-Kante 10 auf, deren Querschnitt sich ausgehend von der Oberflächenebene des Objektträgers zu der am weitesten vorstehenden Stelle der Elektrode verjüngt. Dadurch entsteht beim Anlegen einer Elektroporations-Spannung an das Porationswerkzeug 6 in dem aktiven Elektrodenbereich 8 eine besonders hohe elektrische Feldstärke, die das Öffnen der Zellmembran erleichtert.

Der die Hohl-Elektrode durchsetzende Teilbereich des Durchtrittskanals 7 kann mit Nährmedium 2 gefüllt sein. Dadurch können die elektrischen Ladungsträger von der den Durchtrittskanal 7 umgrenzenden Innenwand der Elektrode zu der an der Mündung des Durchtrittskanals 7 befindlichen Zellmembran gelangen, wodurch sich insgesamt eine größere wirksame Elektrodenfläche ergibt und der elektrische Kontaktwiderstand zwischen der Elektrode und der Zellflüssigkeit entsprechend vermindert ist. Die Elektrodenoberfläche kann eine Oberflächenrauhigkeit aufweisen, welche die Oberfläche der Elektrode vergrößert. Die Elektrode kann beispielsweise aus porösem Silizium bestehen oder eine Beschichtung aus diesem oder einem anderen porösen Material aufweisen.

Wie in Fig.1, 4, 6 und 7 besonders gut erkennbar ist, weist der elektrische Isolator 9 innerhalb des Auflagebereichs 5 einen gegenüber dessen Oberflächenebene vorstehenden Vorsprung 11 auf, an dessen der Oberflächenebene abgewandten freiem Ende der aktive Elektrodenbereich 8 der Elektroporations-Elektrode angeordnet ist. Der aktive Elektrodenbereich 8 ist dadurch elektrisch gut leitend an die an dem Auflagebereich anhaftende Zelle 3 angekoppelt. Der Querschnitt des Vorsprungs 11 verjüngt sich ausgehend von der Oberflächenebene des Auflagebereichs 5 zu der am weitesten vorstehenden Stelle hin. Dadurch sind der Vorsprung 11 und die Elektrode fertigungstechnisch besser herstellbar. Außerdem ermöglicht der sich verjüngende Vorsprung 11 eine gute mechanische Festigkeit. Der Vorsprung 11 kann aber auch einen in seiner Erstreckungsrichtung konstanten Querschnitt aufweisen. Der Vorsprung 11 kann beispielsweise mittels LIGA-Technik hergestellt werden.

Der Objektträger 4 weist ein im wesentlichen plattenförmiges Substrat 12 aufweist, das beispielsweise aus einem Halbleitermaterial (zum Beispiel Silizium oder Gallium-Arsenid), Siliziumcarbid, Glas oder Kunststoff bestehen kann. Auf dieses Substrat kann der Isolator 9 als Beschichtung, beispielsweise durch Sputtern aufgebracht sein. Gegebenenfalls kann das Substrat 12 auch eine flexible Folie sein.

Bei dem Ausführungsbeispiel nach Figur 6 ist zwischen dem Porationswerkzeug 6 und dem Substrat 12 des Objektträgers 4 ein Piezoelement 13 angeordnet, das an seinem freien, relativ zu dem Objektträger 4 beweglichen Ende das Porationswerkzeug 6 trägt. Dieses hat an seinem dem Piezoelement 13 abgewandten Ende eine scharfe Ring-Kante 10, die Gebrauchsstellung an der Zelle 3 angreift. Mit seinem dem Porationswerkzeug 6 abgewandten Ende ist das Piezoelement 13 an dem Substrat 21 des Objektträgers 4 fixiert ist. In dem Auflagebereich 5 ist der das Porationswerkzeug 6 umgrenzende elektrische Isolator 9 angeordnet, der über die Kegelmantelfläche des Porationswerkzeugs 6 bis dicht an deren scharfe Kante 32 herangeführt ist. Mittels des Piezoelements 13 kann die Kante 10 des Porationswerkzeugs 6 etwa in Richtung der Oberflächennormale der durch den Auflagebereich 5 aufgespannten Ebene relativ zu dem Objektträger 4 auf eine an dem Auflagebereich 5 angelagerte Zelle 3 zu und von dieser wegbewegt werden. Das Piezoelements 13 ist dazu mittels elektrischer Anschlußleitungen 14 mit einer Stromversorgung verbindbar. Die Isolator 9 besteht aus einem elastischen Material, das bei einer Ansteuerung des Piezoelements über die Anschlußleitungen 14 eine Relativbewegung zwischen dem Porationswerkzeug 6 und dem Substrat 12 des Objektträgers 4 ermöglicht.

Mit dem Piezoelement 13 können einzelne mechanische Impulse, eine Impulsfolge oder eine mechanische Schwingung auf einen Teilbereich der Zellmembrane übertragen werden.Dabei wird ein etwa kreisscheibenförmiger Bereich der Zellmembran aus dem Membranverbund der Zelle 3 herausgeschnitten. Durch die dadurch in der Zellmembran entstehende Öffnungs kann das Innere der Zelle 3 anschließend manipuliert werden kann.

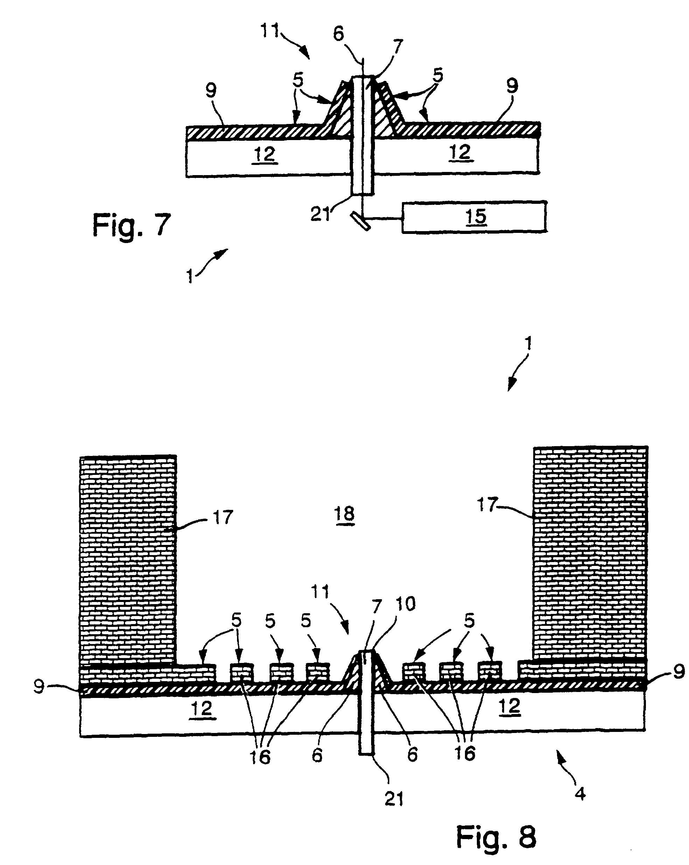

Bei dem Ausführungsbeispiel nach Figur 7 ist das Porationswerkzeug 6 ein Laserstrahl, der durch den Durchtrittskanal 7 in den Auflagebereich 5 einkoppelbar ist. Der Laserstrahl kann mit einem externen Laser 15 oder mittels einer in den Objektträger 4 integrierten Laserdiode erzeugt werden. Wie aus Fig.7 schematisch erkennbar ist, wird der Laserstrahl an einer von der Mündung des Durchtrittskanals 7 beabstandeten Stelle mittels geeigneter Strahlformungs- und/oder -führungsmittel in Erstreckungsrichtung des Durchtrittskanals 7 in diesen eingekoppelt und tritt an der Mündung des Durchtrittskanals 7 etwa in Richtung der Oberflächennormalen der durch den Auflagebereich 5 aufgespannten Ebene aus dem Auflagebereich 5 aus. Mit dem Laserstrahl kann ein Teilbereich der Zellmembran der an dem Auflagebereich 5 angelagerten biologischen Zelle 3 bestrahlt werden. Die Wellenlänge der Laserstrahlung ist so gewählt, daß die Zellmembran die Laserstrahlung absorbiert. Beim Bestrahlen der Zellmembran mit dem Laserstrahl wird diese so stark erwärmt, daß sich die Zellmembran an der bestrahlten Stelle öffnet. Nach dem Einbringen der Öffnung wird die Laserstrahlung abgeschaltet, so daß dann durch die Öffnung hindurch das Zellinnere manipuliert werden kann. Der die Mündung des Durchtrittskanal 7 in dem Auflagebereich 5 umgrenzende Isolator 9 dichtet das Innere der an dem Auflagebereich 5 anhaftenden, geöffneten Zelle gegen das Nährmedium 2 ab.

Bei dem Ausführungsbeispiel nach Figur 8 ist um das freie Ende des gegenüber der Oberflächenebene des Auflagebereichs 5 vorstehenden, die scharfe Kante 10 aufweisenden Porationswerkzeugs 6 herum in einem etwa ringförmigen Bereich eine chemische Substanz immobilisiert, die bei Berührung mit einer an dem Auflagebereich 5 angelagerten Zelle 3 eine Öffnung in deren Zellmembran einbringt.

Diese Ausführungsform weist einen besonders einfachen Aufbau auf.

Bei dem Ausführungsbeispiel gemäß Fig.4 und 5 ist der Durchtrittskanal 7 mit einem Fluid gefüllt. Der Durchtrittskanal 7 ist mit einer Pumpe verbunden, mit dem das in dem Durchtrittskanal 7 enthaltende Fluid gegen eine die Mündung des Durchtrittskanals 7 abdichtende Zellmembrane einer dort angelagerten Zelle 3 mit einem steuerbaren Unterdruck beaufschlagbar ist. Der die Mündung des Durchtrittskanals 7 umgrenzende Rand weist eine ringförmige scharfe Messer-Kante 10 auf, die gegenüber der Oberflächenebene des Auflagebereichs 5 vorsteht. Nach dem Anlagern der Zelle 3 an dem Porationswerkzeug 6 wird ein Teilbereich der Zellmembran der Zelle 3 durch Absaugen von Nährmedium 2 aus dem Durchtrittskanal 7 kurzzeitig so stark mit Unterdruck beaufschlagt, daß der von der scharfen Kante 10 des Porationswerkzeugs 6 umgrenzte Membranbereich der Zellmembran aus dem Membranverbund herausgelöst wird. Dadurch wird eine Öffnung in die Zellmembran eingebracht, durch welche das Innere der Zelle 3 manipuliert werden kann. Nach dem Einbringen der Öffnung wird der Unterdruck in dem Durchtrittskanal 7 abgeschaltet.

Nach dem Einbringen der Öffnung in die Zellmembran der Zelle 3 kann die mit dem Durchtrittskanal 7 verbundene Pumpe kurzzeitig in ihrer Förderrichtung umgekehrt werden, um eine in dem Durchtrittskanal 7 befindliche Substanz, beispielsweise ein Medikament und/oder einen Fluorenzfarbstoff durch die Öffnung der Zellmembran direkt in das Zellinnere zu injizieren. Wenn an dem Porationswerkzeug 6 keine Zelle 3 angelagert ist, kann der Durchtrittskanal 7 außerdem dazu benutzt werden, um in das Nährmedium 2 eine entsprechende Substanz einzuleiten.

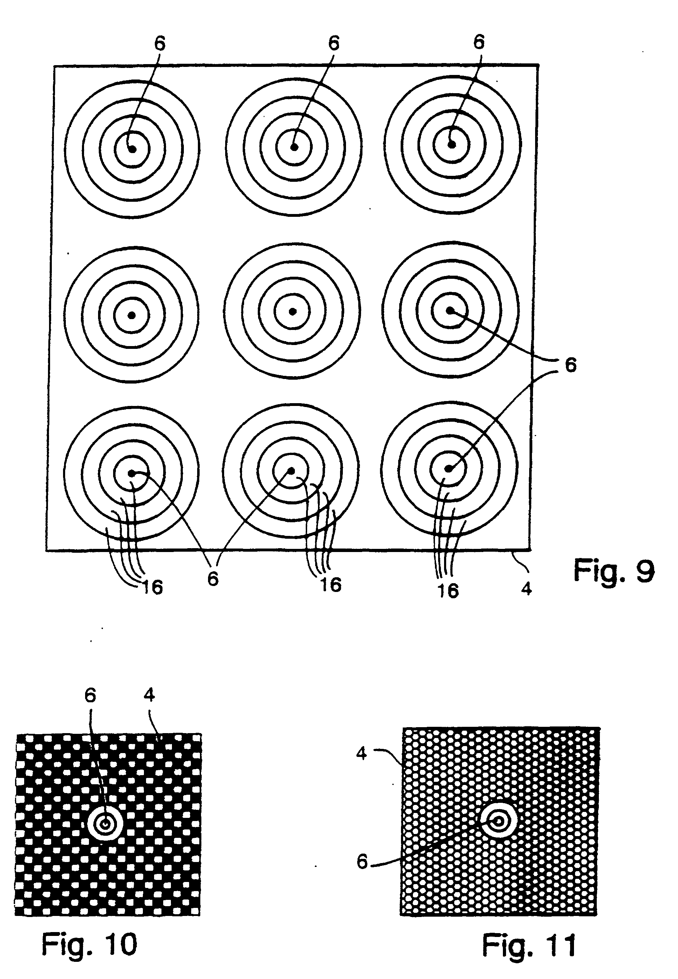

Bei den Ausführungsbeispielen gemäß Fig.8 und 9 weist der Objektträger 4 im Auflagebereich 5 jeweils mehrere, um das Porationswerkzeug 6 umlaufende Profilierungsvertiefungen 16 auf.

Wie aus Fig.1 besonders gut erkennbar ist, wird dadurch die Abdichtung der Zellmembran der Zelle 3 gegen den Auflagebereich 5 des Objektträgers 4 verbessert.

Die Profilierungsvertiefungen 16 sind geschlossene Ringnuten, die konzentrisch zu dem Porationswerkzeug 6 angeordnet sind. Die Ringnuten weisen jeweils einen etwa rechteckförmigen Querschnitt auf. Zueinander benachbarte Ringnuten sind jeweils in etwa gleichen Abständen zueinander angeordnet (Fig. 9). Die Abstände zueinander benachbarter Profilierungsvertiefungen 16 und die Tiefe dieser Profilierungsvertiefungen 16 sind an den Typ der an dem Auflagebereich 5 anzulagernden Zellen 3 angepaßt. Die Kanten der Profilierungsvertiefungen 16 können gerundet sein, um das adhärente Anlagern einer Zelle 3 zu erleichtern.

Die Profilierungsvertiefungen 16 können in ihrem Verlauf Unterbrechungen aufweisen, wie dies am Beispiel einer Karo-Strukturierung in Fig. 10 und einer Wabenstruktur in Fig.11 gezeigt ist. Die Oberflächenprofilierungen, die Oberflächenrauhigkeit und das Oberflächenmaterial können jeweils an einen bestimmten Zelltyp angepaßt sein. Dadurch kann die Zelladhäsion verbessert oder gesteuert werden.

Bei den Ausführungsbeispielen nach Fig.1 und 8 ist die Oberflächen-Profilierung 16 als Beschichtung mit Methoden der Halbleitertechnik auf den elektrischen Isolator 9 aufgebracht. Der Objektträger 4 ist dadurch als Halbleiterchip auf einfache Weise herstellbar. Die Oberflächen-Profilierung kann aber auch mit anderen Verfahren, beispielsweise in Dickschichttechnik auf den Isolator 9 aufgebracht werden.

Bei dem Ausführungsbeispiel gemäß Fig.8 sind beidseits des Porationswerkzeugs 6 Begrenzungswände 17 angeordnet, die zusammen mit dem Isolator 9 einen im Querschnitt etwa U-förmigen Führungskanal 18 bilden. Dabei sind die Profilierungen 16 und das Porationswerkzeug 6 am Boden des Führungskanals 18 zwischen den Begrenzungswänden 17 angeordnet. Die Begrenzungswände 17 bilden ein Hindernis für in dem Führungskanal 18 befindliche Zellen 3, das diese nicht oder nicht ohne weiteres überwinden können. Die Zellen 3 können sich dadurch im wesentlichen nur in Erstreckungsrichtung des Führungskanals 18 bewegen, wobei sie zwangsläufig mit dem Porationswerkzeug 6 in Berührung geraten.

Der lichte Abstand der beidseits des Porationswerkzeugs 6 angeordneten Begrenzungswände 17 ist an die Abmessungen der Zellen 3 angepaßt und ist vorzugsweise etwas größer gewählt als der Zelldurchmesser der Zellen 3. Gegebenenfalls können mehrere Porationswerkzeuge 6 in Erstreckungsrichtung des Führungskanals 18 hintereinander angeordnet sein. Dadurch können mehrere Zellen 3 gleichzeitig geöffnet und intrazellulär manipuliert werden. Der Querschnitt des Führungskanals 18 kann sich in Erstreckungsrichtung verjüngen oder erweitern, d.h. der Führungskanal 18 kann an unterschiedlichen Stellen eine unterschiedliche Breite und/oder unterschiedliche Querschnittsabmessungen aufweisen. Ausgehend von der tiefsten zu der am weitesten vorstehenden Stelle des Führungskanals 18 kann sich der Querschnitt des Führungskanals 18 beispielsweise verjüngen.

Bei dem Ausführungsbeispiel gemäß Fig.9 sind im Auflagebereich 5 des Objektträgers 4 mehrere Porationswerkzeuge 6 in Form eines Arrays angeordnet. Die einzelnen Porationswerkzeuge 6 sind jeweils an Rasterpunkten eines karthesischen Koordinatensystems angeordnet. Die Porationswerkzeuge 6 können aber auch in anderer Weise in dem Auflagebereich 5 verteilt sein, beispielsweise in zueinander versetzten Reihen oder Spalten oder frei verteilt.

Auf und zwischen den Porationswerkzeugen 6 können zur Optimierung des Wachstums der Zellen 3 Leitstrukturen angeordnet sein, die ein gezieltes Anlagern der Zellen 3 auch und/oder zwischen den Porationswerkzeugen 6 ermöglichen. Die Leitstrukturen können beispielsweise eine Oberflächenstrukturierung, eine Beschichtung oder eine entsprechende Topographiegestaltung umfassen. Für unterschiedliche Zelltypen können verschiedene Abstände zwischen zueinander benachbarten Porationswerkzeuge 6 vorgesehen sein.

Bei den in der Zeichnung gezeigten Ausführungsbeispielen ist der Durchtrittskanal 7 jeweils mit einer Pumpe verbunden, mittels der Nährmedium 2 aus dem Auflagebereich 5 des Objektträgers 4 abgesaugt und an anderer Stelle wieder dem Auflagebereich 5 zugeführt werden kann. Dadurch wird das Anlagern einer Zelle 3 an das Porationswerkzeug 6 erleichtert. Gegebenenfalls kann nach dem Anlagern einer mittels des Durchtrittskanal 7 an das Porationswerkzeug 6 angesaugten Zelle noch für eine bestimmte Zeitdauer ein schwacher Unterdruck auf die Zelle 3 ausgeübt werden, bis diese selbständig an dem Auflagebereich 5 anhaftet.

Bei den Ausführungsbeispielen nach Figur 1 bis 8 ist der Objektträger 4 jeweils etwa plattenförmig ausgebildet. Der Durchtrittskanal 7 durchsetzt den Objektträger 4 ausgehend von dem Auflagebereich 5 zu der diesem abgewandten Rückseite des Objektträgers. Bei dem Ausführungsbeispiel nach Figur 1 weist der Objektträger 4 im Bereich des Durchtrittskanals 7 eine Wandungsschwächung 20 auf, die eine geringere Wanddicke hat als ein seitlich dazu benachbarter Wandungsbereich. Dies wird durch eine an der Rückseite des die Wandungsschwächung 20 aufweisenden Objektträgersbereichs angeordnete trichterförmige Einformung 19 erreicht. Der Querschnitt der Einformung 19 verengt sich ausgehend von der rückseitigen Oberflächenebene des Objektträgers 4 zu der tieftsten Stelle der Einformung 19 hin. Dadurch ergibt sich eine gute mechanische Stabilität. An der Rückseite des Objektträgers 4 weist der Durchtrittskanal 7 eine Auschlußstelle 21 zum Verbinden mit einer Pumpe, einem Unter- oder Überdruckreservoir, einem Laser oder dergleichen Einrichtung auf. Erwähnt werden soll noch, daß auch bei den Ausführungsbeispielen nach Fig. 2 bis 11 der Objektträger im Breich des Durchtrittskanals 7 eine reduzierte Wandstärke aufweisen kann.

Zusammenfassend betrifft die vorliegende Erfindung ein Verfahren zur intrazellulären Manipulation einer biologischen Zelle 3, die in einem Nährmedium 2 adhärent an einem Auflagebereich 5 angelagert wird. Innerhalb des Auflagebereichs 5 der Zelle 3 wird mit Abstand von deren Auflagerand eine Öffnung in die Zellmembran der Zelle 3 eingebracht. Dabei dichtet der die Öffnung umgrenzende, an dem Auflagebereich 5 anhaftende Rand der Zellmembran die im Inneren der Zelle 3 befindliche Zellflüssigkeit gegen das Nährmedium 2 ab und isoliert die Zellflüssigkeit gegen das Nährmedium 2. Durch die Öffnung hindurch wird das Innere der Zelle 3 manipuliert.

## Patentansprüche

1. Verfahren zur intrazellulären Manipulation wenigstens einer in einem Nährmedium befindlichen, adhärent an einem Auflagebereich angelagerten biologischen Zelle (3), wobei in die Zellmembran der Zelle (3) eine Öffnung eingebracht und das Zellinnere durch diese Öffnung hindurch manipuliert wird, **dadurch gekennzeichnet, daß** die Öffnung innerhalb des Auflagebereichs (5) der Zelle (3) und mit Abstand von deren Auflagerand in die Zellmembrane eingebracht wird.

2. Verfahren nach Anspruch 1, **dadurch gekennzeichnet, daß** die Öffnung mittels Elektroporation in die Zellmembran eingebracht wird.

3. Verfahren nach Anspruch 1 oder 2, **dadurch gekennzeichnet, daß** zum Einbringen der Öffnung in die Zellmembran auf einen Teilbereich der Zellmembran wenigstens ein mechanischer Impuls ausgeübt wird.

4. Verfahren nach einem der Ansprüche 1 bis 3, **dadurch gekennzeichnet, daß** die Öffnung mittels Schallwellen, insbesondere Ultra- und/oder Hyperschallwellen in die Zellmembran eingebracht wird.

5. Verfahren nach einem der Ansprüche 1 bis 4, **dadurch gekennzeichnet, daß** die Schallwellen auf den zu öffnenden Bereich der Zellmembran fokussiert wird.

6. Verfahren nach einem der Ansprüche 1 bis 5, **dadurch gekennzeichnet, daß** mehrere Schallwellen derart überlagert werden, daß sich ihre Schwingungen in dem zu öffnenden Bereich der Zellmembran zu einer Schwingung mit erhöhter Amplitude überlagern.

7. Verfahren nach einem der Ansprüche 1 bis 6, **dadurch gekennzeichnet, daß** zum Einbringen der Öffnung in die Zellmembran ein Teilbereich der Zellmembran der Zelle (3) mit energiereicher Strahlung, insbesondere mit Laserstrahlung bestrahlt wird.

8. Verfahren nach einem der Ansprüche 1 bis 7, **dadurch gekennzeichnet, daß** die Öffnung durch Einwirkung einer chemischen Porations-Substanz in die Zellmembran der Zelle (3) eingebracht wird.

9. Verfahren nach einem der Ansprüche 1 bis 8, **dadurch gekennzeichnet, daß** eine elektrisch und/oder chemisch und/oder durch Strahlung aktivierbare Porations-Substanz verwendet wird, die zum Einbringen der Öffnung in die Zellmembran durch Einwirkung von Strahlung, einer Chemikalie und/oder eines elektrischen Feldes aktiviert wird.

10. Verfahren nach einem der Ansprüche 1 bis 9, **dadurch gekennzeichnet, daß** ein zu öffnender Teilbereich der Zellmembran durch Beaufschlagung mit einem Unterdruck und/oder einem Überdruck aus dem Membranverband herausgelöst wird.

11. Verfahren nach einem der Ansprüche 1 bis 10, **dadurch gekennzeichnet, daß** die Zelle (3) durch eine Saugkraft an dem Auflagebereich fixiert wird.

12. Verfahren nach einem der Ansprüche 1 bis 11, **dadurch gekennzeichnet, daß** durch die Öffnung hindurch wenigstens eine Substanz und/oder ein Zellbestandteil aus dem Zellinneren entnommen und/oder in das Zellinnere eingebracht wird.

13. Vorrichtung zur Manipulation des Zellinneren wenigstens einer in einem Nährmedium befindlichen, eine Zellmembran aufweisenden biologischen Zelle (3), mit einem Objektträger, der wenigstens einen Auflagebereich (5) hat, an dem die Zelle (3) adhärent anlagerbar ist, mit einem Porationswerkzeug (6) zum Öffnen der Zellmembran, und mit wenigstens einem im Bereich des Porationswerkzeugs (6) befindlichen Durchtrittskanal (7) zur Manipulation des Zellinneren, **dadurch gekennzeichnet, daß** das Porationswerkzeug (6) innerhalb des Auflagebereichs (5) angeordnet ist.

14. Vorrichtung nach Anspruch 13, **dadurch gekennzeichnet, daß** das Porationswerkzeug (6) innerhalb des Auflagebereichs (5) von wenigstens einem elektrischen Isolator (9) umgrenzt ist.

15. Vorrichtung nach Anspruch 13 oder 14, **dadurch gekennzeichnet, daß** das Porationswerkzeug (6) die im Auflagebereich (5) des Objektträgers (4) befindliche Mündung des Durchtrittskanals (7) im wesentlichen konzentrisch umgrenzt.

16. Vorrichtung nach einem der Ansprüche 13 bis 15, **dadurch gekennzeichnet, daß** das Porationswerkzeug (6) eine Elektroporations-Elektrode ist, der eine mit dem Nährmedium (2) in Kontakt bringbare Referenzelektrode zugeordnet ist, und daß die Elektroporations-Elektrode und die Referenzelektrode zum Elektroporieren der Zellmembran der Zelle (3) mit einer elektrischen Spannungsquelle verbindbar sind.

17. Vorrichtung nach einem der Ansprüche 13 bis 16, **dadurch gekennzeichnet, daß** das Porationswerkzeug (6) zum Öffnen der Zellmembran der Zelle (3) mittels wenigstens eines Aktuators, insbesondere eines Piezoelements (13) quer zur Oberfläche des Auflagebereichs (5) relativ zu dem Objektträger bewegbar ist.

18. Vorrichtung nach einem der Ansprüche 13 bis 17, **dadurch gekennzeichnet, daß** der Aktuator mit einer Ansteuereinrichtung zum Erzeugen einer mechanischen Schwingung, insbesondere einer Ultra- oder Hyperschallschwingung verbunden ist.

19. Vorrichtung nach einem der Ansprüche 13 bis 18, **dadurch gekennzeichnet, daß** das Porationswerkzeug (6) mindestens eine, vorzugsweise gegenüber der Oberflächenebene des Auflagebereichs (5) vorstehende scharfe Spitze oder Kante (10) aufweist.

20. Vorrichtung nach einem der Ansprüche 13 bis 19, **dadurch gekennzeichnet, daß** als Porationswerkzeug (6) ein Laserstrahl vorgesehen ist und daß der Strahlengang des Laserstrahls durch den Durchtrittskanal (7) hindurch zu der im Auflagebereich befindlichen Mündung geführt ist.

21. Vorrichtung nach einem der Ansprüche 13 bis 20, **dadurch gekennzeichnet, daß** zum Erzeugen des Laserstrahls eine Laserdiode in den Objektträger (4) integriert ist.

22. Vorrichtung nach einem der Ansprüche 13 bis 21, **dadurch gekennzeichnet, daß** das Porationswerkzeug (6) zum Öffnen der Zellmembran der Zelle (3) im Auflagebereich des Objektträgers (4) eine chemische Porations-Substanz aufweist und/oder daß eine chemische Porations-Substanz durch den Durchtrittskanal (7) zu dessen Mündung zuführbar ist.

23. Vorrichtung nach einem der Ansprüche 13 bis 22, **dadurch gekennzeichnet, daß** sie wenigstens eine Pumpe aufweist, die zum Öffnen der Zellmembran durch Beaufschlagung mit Unteroder Überdruck mit dem Durchtrittskanal (7) verbunden ist und/oder daß in der Durchtrittskanal (7) über ein Ventil oder dergleichen Absperrelement mit einem Unter- oder Überdruckspeicher verbindbar ist.

24. Vorrichtung nach einem der Ansprüche 13 bis 23, **dadurch gekennzeichnet, daß** das Porationswerkzeug (6) an einem gegenüber der Oberflächenebene des Auflagebereichs (5) vorstehenden Vorsprung (11) angeordnet ist.

25. Vorrichtung nach einem der Ansprüche 13 bis 24, **dadurch gekennzeichnet, daß** sich der Querschnitt des Vorsprungs (11) ausgehend von der Oberflächenebene des Auflagebereichs (5) zu der am weitesten vorstehenden Stelle verjüngt.

26. Vorrichtung nach einem der Ansprüche 13 bis 25, **dadurch gekennzeichnet, daß** der Objektträger (4) im Auflagebereich (5) eine Profilierung aufweist, die wenigstens eine um das Porationswerkzeug umlaufende Profilierungsvertiefung und/oder einen um das Porationswerkzeug umlaufenden Prafilierungsvorsprung (16) hat.

27. Vorrichtung nach einem der Ansprüche 13 bis 26, **dadurch gekennzeichnet, daß** die Profilierungsvertiefung und/oder der Profilierungsvorsprung (16) in Erstreckungsrichtung durch wenigstens eine Unterbrechung unterbrochen ist.

28. Vorrichtung nach einem der Ansprüche 13 bis 27, **dadurch gekennzeichnet, daß** die Profilierungsvertiefung und/oder der Profilierungsvorsprung (16) ringförmig ausgebildet ist und daß vorzugsweise mehrere solcher ringförmiger Profilierungsvertiefungen und/oder Profilierungsvorsprünge (16) im wesentlichen konzentrisch zum Porationswerkzeug (6) angeordnet sind.

29. Vorrichtung nach einem der Ansprüche 13 bis 28, **dadurch gekennzeichnet, daß** der elektrische Isolator (9) eine an der Oberfläche der Profilierung angeordnete Isolationsschicht ist.

30. Vorrichtung nach einem der Ansprüche 13 bis 29, **dadurch gekennzeichnet, daß** der (die) Profilierungsvorsprung (-vorsprünge) (16) auf die Oberfläche des elektrischen Isolators (9) aufgebracht ist (sind).

31. Vorrichtung nach einem der Ansprüche 13 bis 30, **dadurch gekennzeichnet, daß** im Auflagebereich (5) des Objektträgers (4) an dessen Oberfläche eine wenigstens ein Zelladhäsionsprotein aufweisende Beschichtung und/oder eine hydrophile Beschichtung und/oder unmittelbar benachbart zu dem Porationswerkzeug eine hydrophobe Beschichtung angeordnet ist.

32. Vorrichtung nach einem der Ansprüche 13 bis 31, **dadurch gekennzeichnet, daß** der Objektträger (4) als etwa plattenförmiges Trägerteil ausgebildet, das an einer Flachseite den Auflagebereich (5) aufweist, daß der Durchtrittskanal (7) den Objektträger (4) ausgehend von dem Auflagebereich (5) zu der diesem abgewandten Rückseite des Objektträgers (4) durchsetzt, und daß der Objektträger im Bereich des Durchtrittskanals (7) eine Wandungsschwächung (20) aufweist, die in Erstreckungsrichtung des Durchtrittskanals (7) eine kleinere Abmessung aufweist als ein zu der Wandungsschwächung benachbarter Wandungsbereich.

33. Vorrichtung nach einem der Ansprüche 13 bis 32, **dadurch gekennzeichnet, daß** die Wandungsschwächung (20) an einer an der dem Auflagebereich (5) abgewandten Rückseite des Objektträgers (4) befindlichen, vorzugsweise trichterförmigen Einformung (19) angeordnet ist.

34. Vorrichtung nach einem der Ansprüche 13 bis 33, **dadurch gekennzeichnet, daß** als mechanische Führung für die Zellen (3) beidseits des Porationswerkzeugs (6) Begrenzungswände (17) angeordnet sind, die vorzugsweise einen nutenartigen Führungskanal (18) begrenzen.

35. Vorrichtung nach einem der Ansprüche 13 bis 34, **dadurch gekennzeichnet, daß** zum Erzeugen eines die Zelle (3) zu dem Porationswerkzeug (6) leitenden elektrischen Feldes im Auflagebereich (5) und/oder benachbart dazu wenigstens eine Zusatzelektrode angeordnet ist.

36. Vorrichtung nach einem der Ansprüche 13 bis 35, **dadurch gekennzeichnet, daß** in dem Auflagebereich (5) mehrere, jeweils wenigstens einen Durchtrittskanal (7) aufweisende Porationswerkzeuge (6) vorzugsweise als Arrays angeordnet sind.

## Claims

1. A process for the intracellular manipulation of at least one biological cell (3) situated in a culture medium and adherently deposited on a support area, whereby an opening is created in the cell membrane of the cell (3) and the interior of the cell is manipulated through this opening,
**characterised in that** the opening in the cell membrane is created inside the support area (5) of the cell (3) and spaced from the edge of its support.

2. A process according to Claim 1,
**characterised in** t hat the opening is created in the cell membrane by means of electroporation.

3. A process according to Claim 1 or 2,
**characterised in that** at least one mechanical impulse is exerted on a portion of the cell membrane to create the opening in the cell membrane.

4. A process according to one of Claims 1 to 3,
**characterised in that** the opening is created in the cell membrane by means of sound waves, in particular ultrasonic and/or hypersonic waves.

5. A process according to one of Claims 1 to 4,
**characterised in that** the sound waves is focussed on the area of the cell membrane to be opened.

6. A process according to one of Claims 1 to 5,
**characterised in that** several sound waves are superimposed in such a manner that their vibrations in the area of the cell membrane to be opened are superimposed to a vibration of increased amplitude.

7. A process according to one of Claims 1 to 6,
**characterised in that** to create the opening in the cell membrane a portion of the cell membrane of the cell (3) is irradiated with energy-rich radiation, in particular laser radiation.

8. A process according to one of Claims 1 to 7,
**characterised in that** the opening is created by the action of a chemical poration substance in the cell membrane of the cell (3).

9. A process according to one of Claims 1 to 8,
**characterised in that** a poration substance that can be activated electrically and/or chemically and/or by radiation is used, which to create the opening in the cell membrane is activated by the action of radiation, a chemical and/or an electrical field.

10. A process according to one of Claims 1 to 9,
**characterised in that** a portion of the cell membrane to be opened is separated from the composite membrane by impinging with an underpressure and/or an overpressure.

11. A process according to one of Claims 1 to 10,
**characterised in that** the cell (3) is fixed on the support area by a suction force.

12. A process according to one of Claims 1 to 11,
**characterised in that** at least one substance and/or one cell constituent is remov ed from the interior of the cell and/or is introduced into the interior of the cell through the opening.

13. An apparatus for the manipulation of the cell interior of at least one biological cell (3) situated in a culture medium and comprising a cell memb rane, having a specimen slide which has at least one support area (5) on which the cell (3) can be adherently supported, having a poration tool (6) for opening the cell membrane, and having at least one through -channel (7) situated in the area of the poration tool (6) for the manipulation of the cell interior,
**characterised in that** the poration tool (6) is disposed inside the support area (5).

14. An apparatus according to Claim 13,
**characterised in that** the poration tool (6) inside the support area (5) is surrounded by at least one electrical insulator (9).

15. An apparatus according to Claim 13 or 14,
**characterised in that** the poration tool (6) substantially concentrically surrounds the opening of the through-channel (7) situated in the support area (5) of the specimen slide (4) .

16. An apparatus according to one of Claims 13 to 15,
**characterised in that** the poration tool (6) is an electroporation electrode (7) with which a reference electrode that can be brought into contact with the culture medium (2) is associated,
and **in that** the electroporation electrode and the reference electrode can be connected to an electric voltage source for the electroporation of the cell membrane of the cell (3) .

17. An apparatus according to one of Claims 13 to 16,
**characterised in that** the poration tool (6) can be moved in relation to the specimen slide to open the cell membrane of the cell (3) by means of at least one actuator, in particular a piezo element (13) at right angles to the surface of the support area (5).

18. An apparatus according to one of Claims 13 to 17,
**characterised in that** the actuator is connected to a control device to generate a mechanical vibration, in particular an ultrasonic or hypersonic sound vibration.

19. An apparatus according to one of Claims 13 to 18,
**characterised in that** the poration tool (6) comprises at least one sharp point or edge (10) preferably protruding in relation to the surface plane of the support area (5).

20. An apparatus according to one of Claims 13 to 19,
**characterised in that** a laser beam is provided as the poration tool (6)
and **in that** the beam path of the laser beam is guided through the through-channel (7) to the opening situated in the support area.

21. An apparatus according to one of Claims 13 to 20,
**characterised in that** a laser diode is integrated into the specimen slide (4) to generate the laser beam.

22. An apparatus according to one of Claims 13 to 21,
**characterised in that** the poration tool (6) for opening the cell membrane of the cell (3) in the support ar ea of the specimen slide (4) comprises a chemical poration substance and/or **in that** a chemical poration substance can be supplied through the through-channel (7) to its opening.

23. An apparatus according to one of Claims 13 to 22,
**characterised in that** it comprises at least one pump which to open the cell membrane by impinging with underpressure or overpressure is connected to the through-channel (7) and/or **in that** in the through -channel (7) it can be connected to an underpressure or overpressure storage device via a valve or a similar shut-off element.

24. An apparatus according to one of Claims 13 to 23,
**characterised in that** the poration tool (6) is disposed at a projection (11) which projections in relation to the surface plane of the support area (5).

25. An apparatus according to one of Claims 13 to 24,
**characterised in that** the cross section of the projection (11) tapers from the surface plane of the support area (5) to the furthest protruding point.

26. An apparatus according to one of Claims 13 to 25,
**characterised in that** the specimen slide (4) in the support area (5) comprises a profile which has at least one profile recess running around the poration tool and/or a profile projection (16) running around the poration tool.

27. An apparatus according to one of Claims 13 to 26,
**characterised in that** the profile recess and/or the profile projection (16) is interrupted in the direction of extension by at least one gap.

28. An apparatus according to one of Claims 13 to 27,
**characterised in that** the profile recess and/or the profile projection (16) has a ring-shaped design
and **in that** preferably several such ring -shaped profile recesses and/or profile projections (16) are disposed substantially concentrically to the poration tool (6).

29. An apparatus according to one of Claims 13 to 28,
**characterised in that** the electrical insulator (9) is an insulating layer disposed on the surface of the profile.

30. An apparatus according to one of Claims 13 to 29,
**characterised in that** the profile projection (p rojections) (16) is (are) mounted on the surface of the electrical insulator.

31. An apparatus according to one of Claims 13 to 30,
**characterised in that** in the support area (5) of the specimen slide (4) on its surface is disposed a coating comprising at least one cell adhesion protein and/or a hydrophilic coating and/or, directly adjacent to the poration tool, a hydrophobic coating.

32. An apparatus according to one of Claims 13 to 31,
**characterised in that** the specimen slide (4) is constructed as a roughly plate-shaped support part which comprises the support area (5) on one flat side,
**in that** the through -channel (7) penetrates the specimen slide (4) starting from the support area (5) to the rear side of the specimen slide (4) further from it,
**and in that** the specimen slide in the region of the through-channel (7) comprises a weakened wall portion (20), which in the direction of extension of the through -channel (7) has a smaller dimension than a wall area adjacent to the weakened wall portion.

33. An apparatus according to one of Claims 13 to 32,
**characterised in that** the weakened wall portion (20) is disposed at a preferably funnel -shaped moulded part (19) situated on the rear side of the specimen slide (4) further from the support area (5).

34. An apparatus according to one of Claims 13 to 33,
**characterised in that** boundary walls (17), which preferably delimit a groove-shaped guide channel (18), are disposed on either side of the poration tool (6) as a mechanical guide for the cells (3).

35. An apparatus according to one of Claims 13 to 34,
**characterised in that** to generate an electric field leading the cell (3) to the poration tool (6) at least one additional electrode is disposed in the support area (5) and/or adjacent thereto.

36. An apparatus according to one of Claims 13 to 35,
**characterised in that** in the support area (5) several poration tools (6) comprising at least one through -channel (7) are preferably disposed as arrays.

## Revendications

1. Procédé pour la manipulation intracellulaire d'au moins une cellule biologique (3) fixée au niveau de la zone de déposition de façon adhérente se trouvant dans un milieu de culture, un orifice étant pratiqué dans la membrane cellulaire de la cellule (3), et la manipulation intracellulaire intervenant par cet orifice,
**caractérisé en ce que**
l'orifice est pratiqué au sein de la zone de charge (5) de la cellule (3) à distance de la bordure de charge dans la membrane cellulaire.

2. Procédé selon la revendication 1,
**caractérisé en ce que**
l'orifice est réalisé par électroporation.

3. Procédé selon la revendication 1 ou 2,
**caractérisé en ce qu'**
au moins une impulsion mécanique est exercée en vue de réaliser l'orifice dans la membrane cellulaire sur une zone partielle de la membrane cellulaire.

4. Procédé selon l'une quelconque des revendications 1 à 3,
**caractérisé en ce que**
l'orifice est réalisé au moyen d'ondes sonores, notamment au moyen d'ondes ultrasonores et/ou hypersonores dans la membrane cellulaire.

5. Procédé selon l'une quelconque des revendications 1 à 4,
**caractérisé en ce que**
les ondes sonores sont focalisées sur la zone de la membrane cellulaire à ouvrir.

6. Procédé selon l'une quelconque des revendications 1 à 5,
**caractérisé en ce que**
plusieurs ondes sonores se superposent, de telle sorte que les variations dans la zone de la membrane cellulaire à ouvrir se chevauchent avec une variation d'amplitude élevée.

7. Procédé selon l'une quelconque des revendications 1 à 6,
**caractérisé en ce qu'**
une zone partielle de la membrane cellulaire de la cellule (3) est exposée à un rayonnement énergétiquement riche, notamment un rayonnement laser, en vue de réaliser l'orifice dans la membrane cellulaire.

8. Procédé selon l'une quelconque des revendications 1 à 7,
**caractérisé en ce que**
l'orifice est réalisé par l'action d'une substance de poration chimique dans la membrane cellulaire de la cellule (3).

9. Procédé selon l'une quelconque des revendications 1 à 8,
**caractérisé en ce qu'**
une substance chimique activable par radiation et/ou de façon chimique et/ou électrique est utilisée, et une substance est activée pour la réalisation de l'orifice dans une membrane cellulaire par action du rayonnement d'un produit chimique et/ou d'un champ électrique.

10. Procédé selon l'une quelconque des revendications 1 à 9,
**caractérisé en ce qu'**
une zone partielle de la membrane cellulaire à ouvrir est séparée du système membranaire par application d'une sous-pression et/ou d'une surpression.

11. Procédé selon l'une quelconque des revendications 1 à 10,
**caractérisé en ce que**
la cellule (3) est fixée par une forte aspiration au niveau de la zone de charge.

12. Procédé selon l'une quelconque des revendications 1 à 11,
**caractérisé en ce que**
une substance et/ou un composant cellulaire est prélevé au sein de la cellule par le biais de l'orifice et/ou est appliqué au sein de la cellule par ledit orifice.

13. Dispositif permettant la manipulation intracellulaire d'au moins une cellule (3) biologique, présentant une membrane cellulaire se trouvant dans un milieu de culture, avec une lame porte-outil qui présente au moins une zone de charge (5), au niveau de laquelle la cellule (3) se fixe de manière adhérente, avec un outil de poration (6) pour ouvrir la membrane cellulaire et avec au moins un canal de pénétration (7) se trouvant dans la zone de l'outil de poration (6),
**caractérisé en ce que**
l'outil de poration (6) est agencé au sein de la zone de charge (5).

14. Dispositif de la revendication 13,
**caractérisé en ce que**
l'outil de poration (6) est délimité au sein de la zone de charge (5) par au moins un isolant électrique (9).

15. Dispositif selon la revendication 13 ou 14,
**caractérisé en ce que**
l'outil de poration (6) délimite l'embouchure se trouvant dans la zone de charge (5) de la lame porte-objet (4) du canal de pénétration (7), essentiellement de façon concentrique.

16. Dispositif selon l'une quelconque des revendications 13 à 15,
**caractérisé en ce que**
l'outil de poration (6) est une électrode d'électroporation, à laquelle est attribuée une électrode de référence pouvant être mise en contact avec le milieu de culture (2), et l'électrode d'électroporation et l'électrode de référence peuvent être reliées à la cellule avec une source de tension électrique, en vue de procéder à l'électroporation de la membrane cellulaire de la cellule (3).

17. Dispositif selon l'une quelconque des revendications 13 à 16,
**caractérisée en ce que**
l'outil de poration (6) peut être déplacé de façon transversale par rapport à la surface de la zone de charge (5) relative à la lame porte-objet en vue de réaliser l'orifice de la membrane cellulaire de la cellule (3) au moyen d'au moins un système de commande, notamment d'un piézo-élément (13).

18. Dispositif selon l'une quelconque des revendications 13 à 17,
**caractérisé en ce que**
le système de commande peut être relié à un dispositif de guidage pour produire des oscillations mécaniques, notamment des oscillations hypersonores ou ultrasonores.

19. Dispositif selon l'une quelconque des revendications 13 à 18,
**caractérisé en ce que**
l'outil de poration (6) présente au moins une aiguille ou une arête aiguë (10) se trouvant au niveau de la surface de la zone de charge (5).

20. Dispositif selon l'une quelconque des revendications 13 à 19,
**caractérisé en ce que**
un rayon laser est prévu comme outil de poration (6), et le tracé du rayonnement du rayon laser est guidé par le canal de pénétration (7), l'embouchure se trouvant dans la zone de charge.

21. Dispositif selon l'une quelconque des revendications 13 à 20,
**caractérisé en ce que**
une diode laser est intégrée dans la lame porte-objet (4) pour générer un rayonnement laser.

22. Dispositif selon l'une quelconque des revendications 13 à 21,
**caractérisé en ce que**
l'outil de poration (6) présente, pour l'ouverture de la membrane cellulaire de la cellule (3), une substance de poration chimique dans la zone de charge de la lame porte-objet (4) et/ou pour qu'une substance de poration chimique puisse être introduite par le canal de pénétration (7) vers son embouchure.

23. Dispositif selon l'une quelconque des revendications 13 à 22,
**caractérisé en ce qu'**
il présente au moins une pompe qui est reliée, en vue de réaliser un orifice dans la membrane cellulaire par charge avec une sous-pression ou une surpression, avec le canal de pénétration (7) et/ou il peut être reliée dans le canal de pénétration (7) au moyen d'un élément de blocage similaire, ou d'une soupape avec un accumulateur de surpression ou de sous-pression.

24. Dispositif selon l'une quelconque des revendications 13 à 23,
**caractérisé en ce que**
l'outil de poration (6) est agencé au niveau d'une saillie existant (11) par rapport au niveau de la surface de la zone de charge (5).

25. Dispositif selon l'une quelconque des revendications 13 à 24,
**caractérisé en ce que**
la coupe transversale de la saillie (11) en partant du niveau de la surface de la zone de charge (5) vers la position existante la plus externe s'amincisse.

26. Dispositif selon l'une quelconque des revendications 13 à 25,
**caractérisé en ce que**
la lame porte-objet (4) présente dans sa zone de charge un profil (5) qui présente au moins une cavité de profil entourant l'outil de poration et/ou une saillie de profil (16) entourant l'outil de poration..

27. Dispositif selon l'une quelconque des revendications 13 à 26,
**caractérisé en ce que**
la cavité de profil et/ou la saillie de profil (16) est interrompue dans la direction de prolongation par au moins une interruption.

28. Dispositif selon l'une quelconque des revendications 13 à 27,
**caractérisé en ce que**
la cavité de profil et/ou la saillie de profil (16) sont constituées sous forme de cercle, et plusieurs de ces cavités de profil en forme de cercle et/ou de ces saillies de profil sont agencées de manière essentiellement concentrique par rapport à l'outil de poration (6).

29. Dispositif selon l'une quelconque des revendications 13 à 28,
**caractérisé en ce que**
l'isolant électrique (9) est une couche isolante agencée à la surface du profil.

30. Dispositif selon l'une quelconque des revendications 13 à 29,
**caractérisé en ce que**
la saillie (les sallies) (16) de profil est (sont) appliquée(s) au niveau de la surface de l'isolant électrique (9).

31. Dispositif selon l'une quelconque des revendications 13 à 30,
**caractérisé en ce qu'**
un revêtement présentant au moins une protéine d'adhésion cellulaire et/ou qu'un revêtement hydrophile et/ou qu'une couche hydrophobe sont agencés dans la zone de charge (5) de la lame porte-objet (4) au niveau de la surface et/ou directement à proximité de l'outil de poration..

32. Dispositif selon l'une quelconque des revendications 13 à 31,
**caractérisé en ce que**
la lame porte-objet (4) est formée comme une partie du substrat en forme de plaque, qui présente au niveau de sa face plane la zone de charge (5), et le canal de pénétration (7) pénètre la lame porte-objet (4) depuis la zone de charge (5) vers la partie arrière de la lame porte-objet (4) orientée vers celui-ci, et la lame porte-objet (4) présente dans la zone du canal de pénétration (7) un affaiblissement de la paroi (20) qui présente dans la direction de prolongation du canal de pénétration (7) une mesure inférieure à celle de la zone de paroi jouxtant l'affaiblissement de la paroi.

33. Dispositif selon l'une quelconque des revendications 13 à 32,
**caractérisé en ce que**
l'affaiblissement de la paroi (20) est agencé au niveau d'un moulage (19) en forme d'entonnoir, se trouvant de préférence au niveau de la partie arrière orientée vers la zone de charge (5) de la lame porte-objet (4).

34. Dispositif selon l'une quelconque des revendications 13 à 33,
**caractérisé en ce que**
les parois de limitation sont agencées comme guide mécanique pour les cellules (3) de part et d'autre de l'outil de poration (6), lesquelles parois (17) délimitent de préférence un canal d'alimentation de type rainure (18).

35. Dispositif selon l'une quelconque des revendications 13 à 34,
**caractérisé en ce que**
pour produire un champ électrique conduisant les cellules (3) vers l'outil de poration (6), une électrode supplémentaire est agencée dans la zone de charge (5) et/ou à proximité de celle-ci au moins.

36. Dispositif selon l'une quelconque des revendications 13 à 35,
**caractérisé en ce que**
dans la zone de charge (5) sont agencés plusieurs outils de poration (6), présentant au moins à chaque fois un canal de pénétration (7), de préférence sous la forme de matrice.
